# DEMANDE DE BREVET EUROPEEN

(11) **EP 1 780 214 A2**
(43) Date de publication de la demande: **02.05.2007**
(21) Numéro de dépôt: 07003419.4
(22) Date de dépôt: 13.06.2001
(51) Int. Cl.: C07H 19/044

(54) **Production combinatoire d'analogues de nucléotides et nucléosides (XiTP)**

(30) Priorité: 14.06.2000 FR 0007557
(62) Demande divisionnaire de: 01945413.1
(71) Demandeur: INSTITUT PASTEUR, 75015 Paris (FR); CENTRE NATIONAL DE LA RECHERCHE SCIENTIFIQUE (CNRS), 75016 Paris (FR)
(72) Inventeur: Marliere, Philippe, 75012 Paris (FR); Pochet, Sylvie, 75012 Paris (FR)
(74) Mandataire: Santarelli

(57) **Abrégé**

La présente invention concerne l'utilisation d'analogues de nucléotides comportant une fonction réactive hydrazide de formule I, notamment le composé de formule suivante : 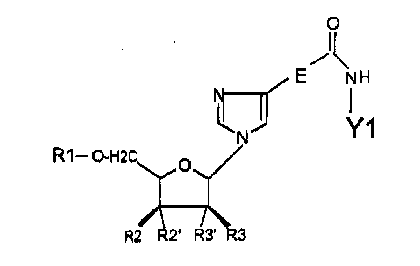 pour induire des mutations ou inhiber une ADN polymérase ou une kinase.

## Description

La présente invention concerne de nouveaux analogues de nucléotides comportant une fonction réactive hydrazide (formule II) servant de synthons de départ pour la préparation de composés (formule I) pouvant induire des mutations ou étant capables d'inhiber une ADN polymérase ou une kinase. L'invention se rapporte également aux acides nucléiques comportant lesdits analogues de nucléosides et nucléotides.

Les inhibiteurs nucléotidiques actuellement disponibles (acyclovir, gancyclovir, AZT, ddC, d4T, 3TC.) présentent des effets secondaires indésirables lors de traitement de longue durée et d'administrations répétées. D'une part, la toxicité intrinsèque de ces analogues de nucléotides provient notamment du fait qu'ils peuvent manquer de spécificité vis-à-vis d'une polymérase ou d'une réverse transcriptase donnée. D'autre part, au bout d'un certain temps, les virus deviennent résistants à ces inhibiteurs même si on augmente les doses.

La synthèse de nouveaux inhibiteurs nucléotidiques représente un enjeu important pour renouveler les méthodes de traitements des infections virales telles que le HIV, CMV et le HSV. Hutchinson, 1990 montre qu'il existe de nombreuses difficultés liées à la synthèse de vastes collections de nucléotides modifiées. Par exemple, il apparaît nécessaire de protéger les fonctions réactives des bases ou des riboses avec toute addition ou modification de groupes.

L'invention permet de pallier ces difficultés grâce à un synthon de base permettant l'obtention en une seule étape d'une librairie d'analogues de nucléotides ou de nucléosides. Grâce à ces librairies, on peut par exemple cribler des inhibiteurs hautement spécifiques de la réverse transcriptase du HIV.

La préparation de nouveaux nucléotides aux appariements ambigus, c'est-à-dire capables de s'incorporer au brin amorce en réponse à plus d'une parmi les quatre bases A, C, G, T dans le brin matrice, s'impose également pour perfectionner les procédés de mutagénèse (Sala et al., 1996; Zaccolo et al., 1996; Pochet et al., 1997). L'agent mutagène ultime consisterait en un monomère de l'ADN qui puisse s'apparier aux quatre bases canoniques au stade de l'incorporation comme triphosphate, puis lors de sa copie comme matrice. Le désoxynucléoside triphosphate d'une telle base ambiguë permettrait de substituer toute base canonique par n'importe laquelle des trois autres bases lors de la réplication par une ADN polymérase. En outre, un procédé d'hypermutagénèse in vivo via l'incorporation de la base ambiguë simplifierait et accélérerait les protocoles d'évolution dirigée des gènes portés par des plasmides, en évitant le recours aux manipulations coûteuses de la PCR erronée. Un résumé justifiant l'emploi de bases ambiguës dans d'autres applications se trouve dans les références Bergstrom et al., 1995; Loakes et al., 1995; Hill et al., 1998.

En outre, la découverte de structures chimiques simplifiées mais douées des mêmes capacités d'appariement univoque que chacune des quatre bases de l'ADN, en d'autres termes des ersatz de A, C, G, T, rend possible la préparation de nouvelles sondes ou amorces nucléiques qui peuvent être utilisées dans diverses techniques d'amplification et de détection d'acides nucléiques cibles.
Par ailleurs, sur le synthon de départ de formule II (voir ci-après), il est possible de greffer des molécules réactives se situant par exemple dans la famille des acides aminés, ce qui permet donc de fonctionnaliser l'ADN.

Des désoxynucléosides portant une purine simplifiée en un simple noyau imidazole différemment substitué aux positions 4 et 5 ont été décrits dans Pochet et al, 1998. Ces nucléosides sont intrinsèquement capables de former des liaisons hydrogène avec les quatre bases canoniques par rotation autour de la liaison glycosidique (conformations syn et anti) et de la liaison carboxamide ("A-like" et "G-like") (Pochet et al., 1995; Pochet et al., 1998). L'incorporation par les ADN polymérases de ces nucléosides (LeBec et al., 1997) et les effets mutagènes de leurs appariements sont décrits dans Sala et al., 1996; Pochet et al., 1997.

Dans le cadre de la présente invention, des groupements latéraux variables peuvent être greffés au monomère de l'ADN simplifié, désigné ci-après par X₀, via un groupe carbohydrazide. Ce synthon de départ permet de préparer de multiples substrats des ADN polymérases en une étape. Le groupe carbohydrazide constitue une fonction très réactive capable de se condenser avec tout aldéhyde ou cétone. Autant de dérivés hydrazones, ci-après désignés par X₁, peuvent donc être formés à partir de X₀. Autant de dérivés hydrazines, ci-après désignés par X₂, peuvent être obtenus par réduction de X₁.

Ainsi, la présente invention porte sur des composés de formule générale I : 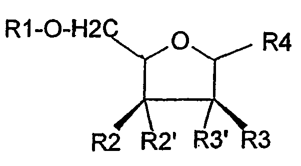 dans laquelle :
- le groupe R1 représente un hydrogène ou un groupe choisi parmi les groupes phosphate, diphosphate ou triphosphate et les groupes protecteurs tels que DMT.
- R2 et R2' sont choisis indépendamment l'un de l'autre parmi H, OH, le phosphoramidite et ses dérivés, le H-phosphonate et un terminateur d'élongation T,
- R3 et R3' sont choisis indépendamment l'un de l'autre parmi H, OH, les halogènes (F, Br, Cl, I), un groupe alkyle linéaire ou ramifié comportant 1 à 6 atomes de carbone et un groupe O-R5, R5 représentant un groupe alkyle linéaire ou ramifié comportant 1 à 6 atomes de carbone,
- R4 représente un hétérocycle (symbolisé par un cercle) substitué, de formule 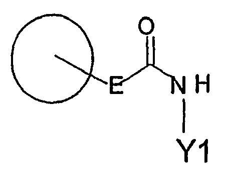
dans lesquelles E désigne un bras espaceur constitué par une chaîne carbonée de 0 à 20 atomes de carbones saturée ou non, comprenant ou non des hétéroatomes et/ou éventuellement substitué ; ledit hétérocycle étant sélectionné parmi :
a) les hétérocycles à 5 atomes de la formule : 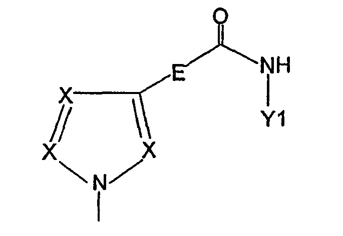 dans laquelle X représente indépendamment les un des autres un groupe CH, C-R6, ou N, R6 étant choisi parmi les halogènes (F, Br, Cl, I), un groupe alkyle linéaire ou ramifié comportant 1 à 6 atomes de carbone et un groupe O-R7 ou S-R7, R7 représentant un hydrogène ou un groupe alkyle linéaire ou ramifié comportant 1 à 6 atomes de carbone,
   lesdits hétérocycles appartenant notamment à la famille des azoles, tels que les cycles 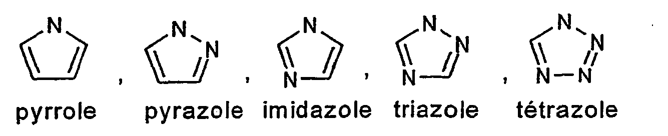 de préférence 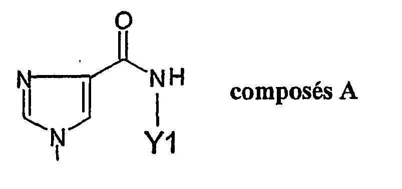
b) les cycles à 6 atomes, notamment les pyrimidines de formule : 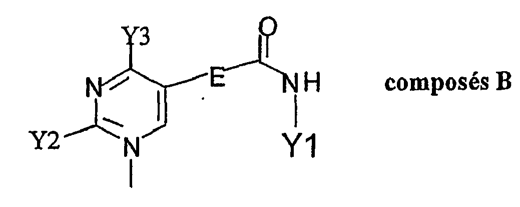
c) les analogues de purines de formule : 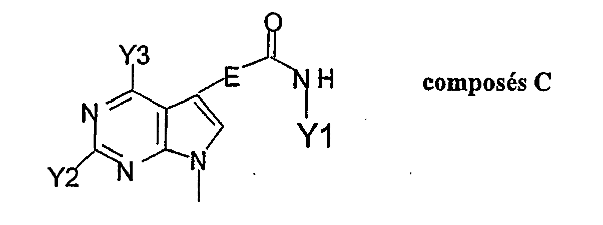
dans lesquels :
- Y1 représente NH₂ (désigné par le synthon X₀) ou un groupe : 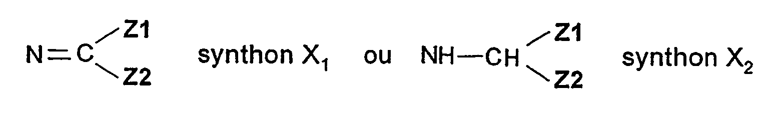
- Y2 et Y3 sont choisis indépendamment l'un de l'autre parmi H, OH, O, NH₂, les halogènes (F, Br, Cl, I), SCH₃, SH, une amine, un amide (dans le sens -NH-CO-R) ou un alkyle linéaire (sens -OR) ou ramifié comportant 1 à 6 atomes de carbone,
   Z1 et Z2 étant choisis indépendamment l'un de l'autre parmi H et un groupe organique.

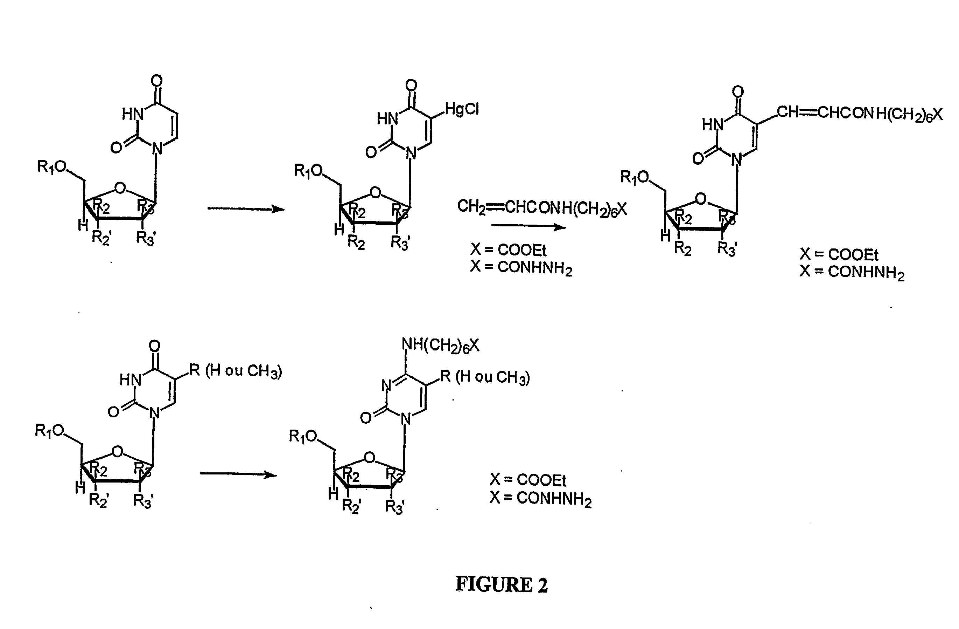

Concernant les termes « purines » et « pyrimidines », on se référera à la définition donnée dans Oxford Dictionary of Biochemistry and Molecular Biology, Oxford Press, 1997. Des exemples de voies de synthèse de ces composés sont décrits aux pages 546 et 547 de ce document.
Concernant les composés B, l'exemple de synthèse présenté à la figure 2 est valable pour les nucléosides en série ribo, desoxy et pour les dérivés phosphorylés. Lorsque X est CONHNH₂ , on obtient un synthon X₀ selon l'invention comprenant un bras espaceur CH=CHCONH(CH2)n, où est n=6 dans cet exemple.

Parmi les synthons X₀, X₁ et X₂, l'invention vise notamment des analogues de nucléosides et des analogues de nucléotides triphosphates que l'on désignera par X₀TP, X₁TP et X₂TP. Les analogues de nucléotides selon l'invention seront de préférence utilisés lorsque l'objectif implique leur incorporation dans un acide nucléique. On utilisera de préférence un analogue de nucléoside selon l'invention pour inhiber une polymérase *in situ* dans une cellule, notamment pour la fabrication d'un médicament.

Dans les composés de formule I, le groupe R1 représente de préférence un groupe triphosphate ou un hydrogène en fonction de l'objectif évoqué ci-dessus.
De même selon l'objectif recherché, au moins un des groupes R2 et R2' peut représenter un groupe OH, ce qui permet la poursuite de l'élongation par une polymérase ou un groupe terminateur T dans le cas où on souhaite obtenir des inhibiteurs des polymérases. Ainsi, au moins un des groupes R2 et R2' peut représenter le groupe T, qui est sélectionné de préférence parmi F, Br, Cl, I, N₃, et un groupe alkyle linéaire ou ramifié comportant 1 à 6 atomes de carbone. Bien entendu, tout groupe équivalent fonctionnant comme un terminateur d'élongation est visé pour la préparation de composé inhibant les réverses transcriptases de rétrovirus.

Avantageusement, lorsque Y1 est NH₂, l'invention a pour objet les synthons de départ de formule générale II (synthon Xo) : 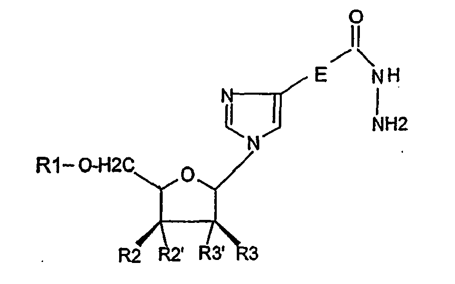 dans lesquels R1, R2, R2', R3, R3' et E correspondent aux groupes de la formule I. Parmi de tels composés, l'invention vise notamment le composé appelé dXoTP dans lequel R1 est un groupe triphosphate, et R3 et R3' représentent H,

Les composés de formule II explicités ci-dessus peuvent être utilisés comme synthon de départ pour la préparation de librairie d'analogues de nucléotides et/ou de nucléosides. Leur groupe carbohydrazide permet en effet de conjuguer en une seule étape ces composés avec toute molécule comportant une fonction aldéhyde ou cétone donnant les synthons X₁ après condensation ou X₂ après réduction.

Par condensation des composés de formule II (synthons X₀ et X₀TP) avec toute molécule comportant une fonction aldéhyde ou cétone, on obtient un composé de formule I dans lequel le groupe Y1 représente la formule générale : 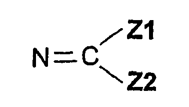 Z1 et Z2 représentant indépendamment l'un de l'autre un hydrogène ou un groupe organique. Ces composés seront désignés par X₁TP (analogues de nucléotide triphosphate) ou X₁ (analogues de nucléosides).
On peut réduire de tels composés et obtenir les composés de formule I (synthons X₂ et X₂TP) dans lequel le groupe Y1 représente la formule générale : 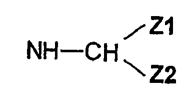 On peut citer par exemple, les composés dans lesquels au moins un des groupes Z1 et Z2 est sélectionné parmi les groupes aromatiques, notamment parmi les groupes de formule :

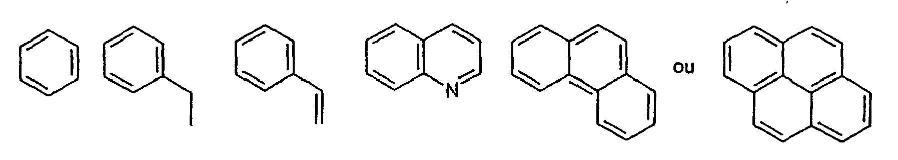

Au moins un des groupes Z1 et Z2 peut également être sélectionné parmi les groupes thiol, alkyle, carbonyle, amine, alcool, aryle, et acide aminé.

Dans un autre aspect, les composés de l'invention peuvent être caractérisés en ce que Z1 ou Z2 forme un cycle avec Y3.

Dans un mode particulier de réalisation, l'invention concerne les composés précités dans lesquels Z1 ou Z2 comprend un marqueur fluorescent ou phosphorescent, notamment la fluorescamine ou la fluorescéine. Par exemple, on peut faire réagir la fluorescamine pour marquer le composé ou bien pour détecter sa présence au sein d'un acide nucléique.

On peut donc préparer des composés avec un bras espaceur et un marqueur, par exemple un composé de formule:

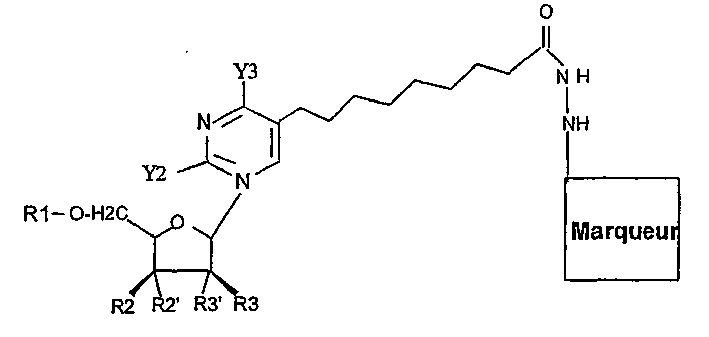

Avantageusement, les composés précités sont des substrats d'une polymérase et peuvent former des appariements avec les bases naturelles, d'une réverse transcriptase et/ou d'une nucléotide kinase.
De plus, lesdits composés peuvent introduire des mutations dans un acide nucléique ou bloquer la synthèse d'ADN ou d'ARN, notamment par les ADN polymérases, les réverses transcriptases de rétrovirus et les kinases. Parmi les kinases, on entend désigner toute kinase capable de phosphoryler les nucléotides mono ou di-phosphate *in vivo* ou *in vitro.* On peut citer plus particulièrement les désoxycytidine kinases, notamment la DCK2 humaine décrite dans US 5,914,258, les désoxyguanosine kinases et les thymidine kinases.

Ainsi, on peut utiliser un composé selon l'invention dans lequel Y1 est NH₂ comme synthon de départ pour la préparation de librairies d'analogues de nucléotides et/ou de nucléosides, et identifier parmi lesdits analogues ceux qui peuvent induire des mutations et/ou bloquer la synthèse d'ADN ou d'ARN.

L'invention se rapporte également à un procédé de préparation de composés de formule générale II décrits précédemment caractérisé en ce qu'il comprend les étapes suivantes :
a) préparation du nucléoside ayant pour hétérocycle un hétérocycle à 5 atomes de formule : 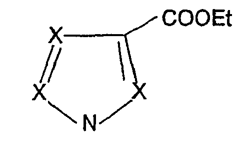 dans laquelle X représente indépendamment les un des autres un groupe CH, C-R6, ou N, R6 étant choisi parmi les halogènes (F, Br, Cl, I), un groupe alkyle linéaire ou ramifié comportant 1 à 6 atomes de carbone et un groupe O-R7 ou S-R7, R7 représentant un hydrogène ou un groupe alkyle linéaire ou ramifié comportant 1 à 6 atomes de carbone,
   lesdits hétérocycles appartenant notamment à la famille des azoles, tels que les cycles 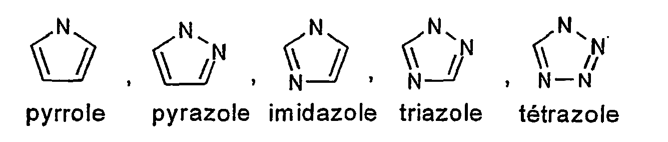
b) protection de l'alcool en 5' et transformation de la fonction ester éthylique en fonction carbohydrazide par l'action de l'hydrazine,
c) protection de la fonction amine du groupe carbohydrazide par un groupe protecteur tel que le groupe benzyloxycarbonyle, acétylation de l'alcool en 3',
d) phosphorylation après déprotection de l'alcool en 5',
e) et hydrogénolyse du groupe benzyloxycarbonyle.

De préférence, l'étape a) est réalisée au moyen d'une N-transdésoxyribosylase dans le cas de désoxyribose et l'étape d) consiste en la phosphorylation du nucléoside par le cyanoéthyl-phosphate en présence de dicyclohexylcarbodiimide; libération concomitante en milieu basique de l'alcool 3' et du groupe cyanoéthyle du 5' phosphate ; puis condensation de pyrophosphate au 5' phosphate activé sous forme de morpholidate.

A partir des composés de formules générales II, on peut préparer un composé de formule générale I décrit ci-dessus par condensation d'un composé de formule générale II avec un aldéhyde ou une cétone, suivi éventuellement d'une réduciton. Il est possible de faire réagir un ou plusieurs composé(s) de formule générale II avec une librairie d'aldéhydes et/ou de cétones, suivie éventuellement d'une réduction, de sorte à obtenir une librairie de composés de formule I.

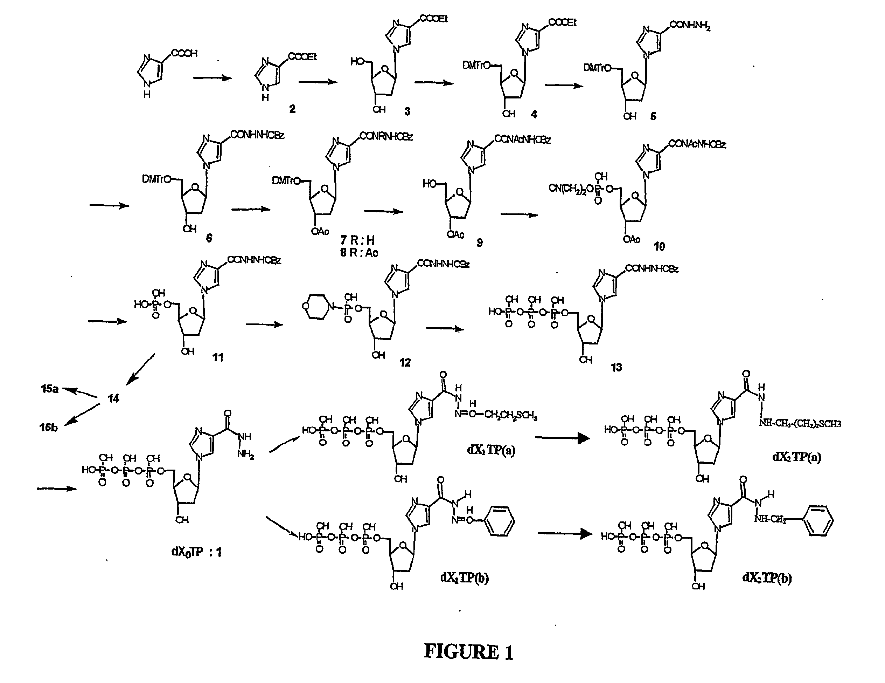

La voie de synthèse décrite dans la figure 1 illustre un mode de réalisation particulier du procédé selon l'invention. L'ester éthylique de l'acide imidazole-4-carboxylique fut converti en son désoxynucléoside par action de la N-transdésoxyribosylase, utilisée sans purification sous forme d'un extrait protéique de Lactobacillus leichmannii, en prenant la thymidine comme source de désoxyribose. Après protection de l'alcool en 5' par un groupe diméthoxytrityle, le nucléoside portant la fonction ester éthylique fut à son tour converti en son dérivé carbohydrazide par action de l'hydrazine. La fonction amine du groupe carbohydrazide fut ensuite protégée par condensation d'un groupe benzyloxycarbonyle. Après acétylation de l'alcool en 3' et libération de l'alcool en 5', le synthon obtenu fut soumis aux étapes de phosphorylation suivantes, à savoir : phosphorylation par le cyanoéthyl-phosphate en présence de dicyclohexylcarbodiimide ; libération concomitante en milieu basique de l'alcool 3' et du 5' phosphate ; condensation de pyrophosphate au 5' phosphate activé sous forme de morpholidate. La production du synthon réactif désoxynucléoside triphosphate du carbohydrazide dX₀TP fut achevée par hydrogénolyse du groupe benzyloxycarbonyle.

La condensation du carbohydrazide dX₀TP avec Z1-CHO et Z1-CO-Z2 conduit aux dérivés hydrazones dX₁TP, puis aux composés dX₂TP après réduction.

Un autre aspect de l'invention se rapporte aux composés susceptibles d'être obtenus à partir des procédés explicités ci-dessus et aux librairies de composés de formule générale I susceptibles d'être obtenues lorsqu'on fait réagir un ou plusieurs composés de formule générale II avec une librairie d'aldéhydes et/ou de cétones.

L'invention concerne également un procédé d'identification de composés capables d'introduire une mutation dans un acide nucléique comprenant les étapes consistant à i) incorporer dans un oligonucléotide synthétique un composé de formule I dans lequel Y1 est NH₂, ii) faire réagir ledit composé par condensation avec au moins un aldéhyde et/ou une cétone, éventuellement suivie d'une réduction, iii) répliquer ledit oligonucléotide à l'aide d'une polymérase et déterminer si une mutation a été introduite dans le brin nouvellement synthétisé.

Ainsi, dans un autre mode de réalisation, l'invention se rapporte à un procédé de mutation ponctuelle d'un acide nucléique caractérisé en qu'on i) prépare un oligonucléotide synthétique comprenant au moins un composé selon l'invention, ii) on réplique ledit oligonucléotide à l'aide d'une polymérase.
Dans un autre alternative, l'invention vise un procédé de mutation aléatoire d'un acide nucléique comprenant les étapes consistant à utiliser un mélange réactionnel comprenant au moins un composé précité pour l'amplification d'un acide nucléique.
Un tel procédé de mutagénèse aléatoire est particulièrement utile pour modifier l'activité d'un polypeptide d'intérêt.

Pour l'élongation, la réplication et l'amplification, on peut utiliser une ADN polymérase exo- telle que par exemple la Taq polymérase, le fragment de klenow et la Vent polymérase.
Toutefois, il est possible d'utiliser des ADN pol exo+ dans le cas où l'on souhaite cribler des composés selon l'invention qui seraient résistants à l'activité exonucléasique. De tels composés sont particulièrement utiles dans les procédés de détection de mutations.

Un aspect supplémentaire concerne un procédé d'identification d'un composé capable d'inhiber une enzyme sélectionnée parmi une polymérase, notamment une réverse transcriptase, et/ou une kinase, notamment toute kinase capable de phosphoryler les nucléotides mono ou di-phosphate *in vivo* ou *in vitro* (désoxycytidine kinases, désoxyguanosine kinases et thymidine kinases) caractérisé en ce que l'on teste l'activité de ladite enzyme en présence d'au moins un composé selon l'invention.

Dans ce procédé, on peut tester l'effet d'au moins un desdits composés sur des cellules infectées par un rétrovirus, ledit composé se trouvant sous la forme d'un analogue de nucléoside. En effet, les nucléosides peuvent traverser la membrane des cellules, ce qui n'est pas le cas des nucléotides à cause de la charge négative des groupes phosphates. On peut donc effectuer un criblage à grande échelle en utilisant une librairie selon l'invention et par un processus itératif avec des fractions de la librairie, on arrive par des expériences de routine à l'identification d'un ou plusieurs composé(s) bloquant la réplication des virus.
L'invention vise les composés susceptibles d'être obtenus à partir du procédé précité.

Par ailleurs, les composés de l'invention peuvent être utilisés dans des amorces ou sondes pour l'amplification et/ou pour la détection d'un acide nucléique cible.
Une « sonde » ou une « amorce » se définit, dans le sens de l'invention, comme étant un fragment nucléotidique comprenant par exemple de 10 à 100, notamment de 15 à 35 nucléotides naturels ou modifiés, comprenant au moins un composé répondant à la formule I décrite ci-dessus et possédant une spécificité d'hybridation dans des conditions déterminées pour former un complexe d'hybridation avec un acide nucléique cible. Les sondes selon l'invention, qu'elles soient spécifiques ou non-spécifiques, peuvent être immobilisées, directement ou indirectement, sur un support solide ; on parle alors de «sonde de capture». Par ailleurs, lesdites sondes peuvent porter un agent marqueur permettant leur détection ; on parle alors de « sonde de détection ».

Une «sonde de capture» est immobilisée ou immobilisable sur un support solide par tout moyen approprié, par exemple par covalence, par adsorption ou par synthèse directe sur un support solide. Ces techniques sont notamment décrites dans la demande de brevet WO 92/10092. La méthode la plus générale consiste à immobiliser l'acide nucléique extrait des cellules de différents tissus ou de cellules en culture sur un support (tels que la nitrocellulose, le nylon^{™}, le polystyrène) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde).

Une « sonde de détection » peut être marquée au moyen d'un marqueur choisi par exemple parmi les isotopes radioactifs, des enzymes, en particulier des enzymes susceptibles d'agir sur un substrat chromogène, fluorigène ou luminescent (notamment une peroxydase ou une phosphatase alcaline), des composés chimiques chromophores, des composés chromogènes, fluorigènes ou luminescents, des analogues de base nucléotitiques, et des ligands tels que la biotine. Le marquage des amorces ou des sondes obtenues selon l'invention est réalisé par des éléments radioactifs ou par des molécules non radioactives. Les entités non radioactives sont sélectionnées parmi les ligands tels la biotine, l'avidine, la streptavidine, la dioxygénine, les haptènes, les colorants, les agents luminescents tels que les agents radioluminescents, chémiluminescents, bioluminescents, fluorescents (fluorescéine isothiocyanate FITC, R-phycoérythrine PE, Quantum Red^{™}, SIGMA et fluorescamine, Molecular Bioprobes) et phosphorescents.

Dans un mode de réalisation particulier, l'invention porte sur les composés de formule I marqués. Comme indiqué précédemment, on peut condenser un composé de formule II avec une molécule comportant une fonction aldéhyde ou cétone. Dans ce cas, lesdites molécules portent un marqueur du type chromophores, composés chromogènes, fluorigènes ou luminescents ou ligands tels que la biotine.

Les acides nucléiques comportant au moins un composé de formule I sont visés par la présente invention. Ils peuvent être mis en oeuvre dans les techniques du type PCR (Erlich, 1989 ; Innis et al., 1990, et Rolfs et al., 1991). Cette technique nécessite le choix de paires d'amorces oligonucléotidiques encadrant le fragment qui doit être amplifié. On peut, par exemple, se référer à la technique décrite dans le brevet américain U.S. N° 4,683,202. D'autres techniques d'amplification peuvent être avantageusement employées comme alternative à la PCR (PCR-like) à l'aide de couple d'amorces selon l'invention. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien connues de l'homme du métier.

L'invention concerne également un kit pour l'amplification et/ou la détection d'un acide nucléique cible et une composition pharmaceutique comprenant un composé selon l'invention ou un acide nucléique selon l'invention. Elle porte également sur l'utilisation d'un acide nucléique décrit ci-dessus comme ribozyme. On entend par « ribozyme » dans le sens de l'invention, un acide nucléique, un dérivé d'acide nucléique ou un hybride chimique entre acide nucléique et peptide qui présente des propriétés catalytiques, de modulation de l'activité ou de l'expression d'une protéine, d'un polypeptide, d'un peptide ou d'un gène. Un acide nucléique selon l'invention peut donc se substituer à un enzyme.

Dans un autre aspect, l'invention se rapporte à des oligonucléotides antisens comprenant au moins un composé de formule I. Par antisens, on entend désigner un oligonucléotide complémentaire d'un ADN ou ARN cible bloquant la transcription ou la traduction. Des exemples d'application sont donnés notamment dans WO9954463, WO9838204, WO9829448, W09735960, WO9710840 et WO9625497.
Dès lors, ledit acide nucléique selon l'invention est utile comme médicament et dans des méthodes de traitement.

L'invention porte également sur l'utilisation d'un composé décrit précédemment pour la fabrication d'un médicament, notamment pour la fabrication d'un médicament destiné au traitement des infections rétrovirales et du cancer.

### Exemple 1 : Procédé de préparation de composés préférés selon l'invention (figure 1).

La synthèse du triphosphate 1 implique l'introduction d'un groupe temporaire de protection sur la fonction hydrazine qui peut être libérée dans des conditions neutres. A cet effet, on a sélectionné le groupe benzyloxycarbonyle. La chaîne triphosphate a été introduite en 3 étapes, conformément aux procédures décrites par Tener et Moffatt. Le nucléoside 3 a été obtenu à partir du composé 2 par transglycosylation enzymatique en utilisant la N-désoxyribosyltransferase comme décrit précédemment par Pochet et al. 1995.
La 5'-dimethoxytritylation du composé **3** dans la pyridine a permis l'obtention du composé **4** avec un rendement de 73 %. Le traitement du composé **4** avec un large excès d'hydrate d'hydrazine à 60° C a conduit au composé **5** qui peut être utilisé dans les étapes suivantes sans purification supplémentaire.
Le groupe benzyloxycarbonyle a été introduit en faisant réagir le composé **5** avec le benzyloxycarbonylsuccinimidate conduisant au composé **6** en un rendement de 81 %. L'acylation avec l'anhydride acétique dans la pyridine produit un mélange de deux composés majeurs correspondant au composé 3'-O-acétylé 7 (24 %) et au composé 3'-O, N-diacétylé **8** (38 %). L'acétylation complète a été rapidement obtenue dans l'acétonitrile en utilisant l'anhydride acétique (2.2 eq.) en présence de triethylamine catalysée par le DMAP permettant d'obtenir le composé 8 avec un rendement de 80 %. Après détritylation du composé **8,** la condensation du composé 9 avec le cyanoéthylphosphate dans la pyridine en présence de DCC, suivie par un traitement avec une solution de 2 % de méthylate de sodium dans le méthanol, le procédé a conduit au composé 5'monophosphate **11** (52 %). Le composé triphosphate **13** a été obtenu en deux étapes en passant par le composé morpholidate **12** avec un rendement de 50 %. La suppression du groupe protecteur benzyloxycarbonyle a été accomplie par hydrogénolyse sur Pd/C en présence de H₂. Ainsi, le traitement des composés **11** et **13** a permis d'obtenir les dérivés monophosphate **14** et triphosphate **1.**

### Exemple 2 : Condensation du composé 1 avec des aldéhydes.

La dérivatisation de la fonction hydrazine a été effectuée en utilisant des aldéhydes aromatiques et aliphatiques : premièrement, le composé monophosphate **14** a été traité avec le 3-méthylthiopropionaldéhyde et le benzaldéhyde dans H₂O/méthanol pour donner les composés **15a** et **15b.** Ces produits ont été isolés par HPLC en phase inverse et ont été caractérisés par la technique de spectrométrique de masse et par RMN. De la même manière, le composé 1 dans le tampon TEAA (pH 7,5) a été traité à 4°C avec un excès d'aldéhydes dans du méthanol pour donner les composés **16a** et **16b.** La structure des dérivés triphosphates a été confirmée par spectrométrie de masse.

On a rajouté au composé 1 dans 0,2 ml de 0,1M TEAA un aldéhyde (25µL dans 0,1 mL de méthanol). Après 2 heures, la solution a été concentrée et purifiée par HPLC à λ=230 nm (0 à 25% acétronitrile dans 10 mM TEAA). Les fractions contenant le produit pur ont été lyophilisées et passées à travers une colonne échangeuse de cations (Dowex) pour donner les triphosphates sous la forme de sels de sodium.

### Exemple 3: Incorporation des analogues dX₀TP et dX₁TP dans les acides nucléiques.

Les analogues (dX₀TP, dX₁TP(a) et dX₁TP(b)) ont été soumis à des expériences d'élongation d'amorce catalysées par le fragment de Klenow exo-. Une amorce a été marquée à son extrémité 5' par réaction d'un [γ-³²P]ATP utilisant la T4 polynucléotide kinase. Les matrices appropriées et les amorces marquées ont été incubées à 75°C pendant 15 minutes, puis refroidies lentement à température ambiante pendant 1 heure. Dans différents mélanges réactionnels, on a réalisé l'élongation de l'amorce avec une ADN polymérase en présence des dX₁TP donnés. Les échantillons ont ensuite été dénaturés puis chargés sur un gel de polyacrylamide (20% 7M d'urée). Après électrophorèse, les gels ont été visualisés par autoradiographie ou à l'aide d'un Phosporimager^{™}.

### Références

Bergstrom D.E., Zhang P., Toma P.H., Andrews P.C. & Nichols R. (1995) "Synthesis, structure, and deoxyribonucleic acid sequencing with a universal nucleoside: 1-(2'-deoxy-β-D-ribofuranosyl)-3-nitropyrrole" J. Am. Chem. Soc. 117, 1201-1209.
Hill F., Loakes D. & Brown D.M. (1998) "Polymerase recognition of synthetic oligonucleotides incorporating degenerate pyrimidine and purine bases" Proc. Nat. Acad. Sci., 95, 4258-4263.
Hutchinson D.W. (1990) TIBTECH, 8, 348-353.
Le Bec C., Roux P., Buc H. & Pochet S. (1997) Derivatives of imidazole-4-carboxamide as substrates for various DNA polymerases. Nucleosides & Nucleotides 16 (7-9), 1301-1302.
Loakes D., Brown D.M., Linde S. & Hill F. (1995) "3-Nitropyrrole and 5-nitroindole as universal bases in primers for DNA sequencing and PCR" Nucleic Acids Research 23, 2361-2366.
Pochet S., Dugué L., Meier A. & Marlière P. (1995) "Enzymatic synthesis of 1-(2-deoxy-β-D-ribofuranosyl)-imidazole-4-carboxamide, a simplified DNA building block" Bioorganic & Medicinal Chemistry Letters 5, 1679-1684.
Pochet S., Dugué L., Sala M., Pezo V. & Wain-Hobson S. (1997) "Ambiguous base pairing of the purine analogue 1-(2-deoxy-β-D-ribofuranosyl)-imidazole-4-carboxamide during PCR" Nucleosides & Nucleotides 16 (7-9), 1749-1752.
Pochet S. & Dugué L. (1998) "Imidazole-4-carboxamide and triazole-4-carboxamide deoxynucleosides as simplified DNA building-blocks with ambiguous base pairing capacity" Nucleosides & Nucleotides 17(9-11), 2003-2009.
Sala M., Pezo V., Pochet S. & Wain-Hobson S. (1996) "Ambiguous base pairing of the purine analogue 1-(2-deoxy-β-D-ribofuranosyl)-imidazole-4-carboxamide during PCR" Nucleic Acids Research 24, 3302-3306.
Zaccolo M., Williams D.M., Brown D.M. & Gherardi E. (1996) "An approach to random mutagenesis of DNA using mixtures of triphosphate derivatives of analogues" J. Mol. Biol. 255, 589-603.

## Revendications

1. Utilisation d'un composé de formule générale I : 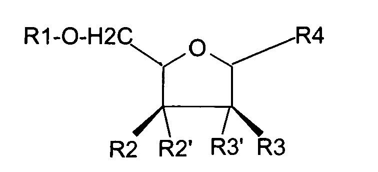 dans laquelle :
- le groupe R1 représente l'hydrogène ;
- R2 et R2' sont choisis indépendamment l'un de l'autre parmi H, OH, le phosphoramidite et ses dérivés, le H-phosphonate et un terminateur d'élongation T,
- R3 et R3' sont choisis indépendamment l'un de l'autre parmi H, OH, les halogènes (F, Br, Cl, I), un groupe alkyle linéaire ou ramifié comportant 1 à 6 atomes de carbone et un groupe O-R5, R5 représentant un groupe alkyle linéaire ou ramifié comportant 1 à 6 atomes de carbone,
- R4 représente un hétérocycle (symbolisé par un cercle) substitué, de formule 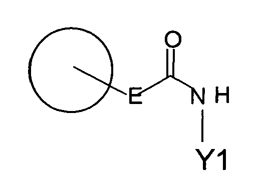
dans lesquelles E désigne un bras espaceur constitué par une chaîne carbonée de 0 à 20 atomes de carbones saturée ou non, comprenant ou non des hétéroatomes et/ou éventuellement substitué ; ledit hétérocycle étant choisi parmi :
c) les hétérocycles à 5 atomes de la formule : 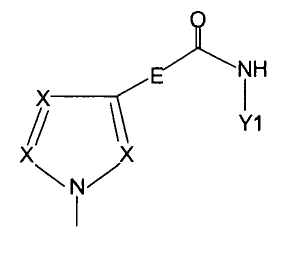 dans laquelle X représente indépendamment les un des autres un groupe CH, C-R6, ou N, R6 étant choisi parmi les halogènes (F, Br, Cl, I), un groupe alkyle linéaire ou ramifié comportant 1 à 6 atomes de carbone et un groupe O-R7 ou S-R7, R7 représentant un hydrogène ou un groupe alkyle linéaire ou ramifié comportant 1 à 6 atomes de carbone, lesdits hétérocycles appartenant notamment à la famille des azoles, tels que les cycles 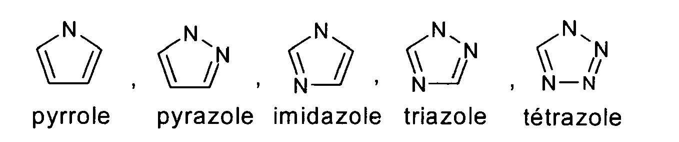 de préférence 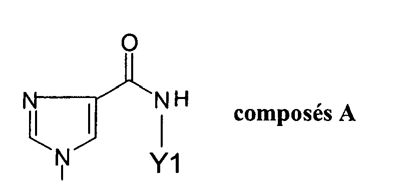
b) les cycles à 6 atomes, notamment les pyrimidines de formule : 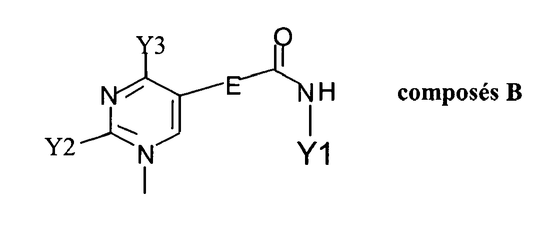
c) les analogues de purines de formule : 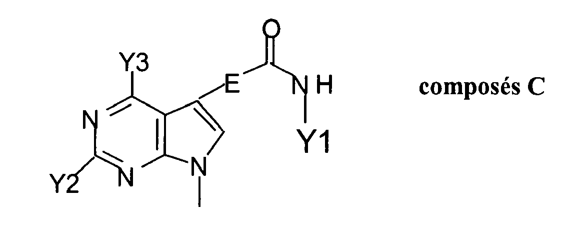
- Y1 représentant NH₂ (désigné par le synthon X₀) ou un groupe : 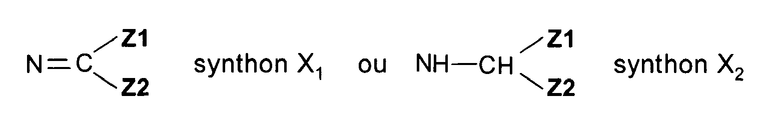
- Y2 et Y3 sont choisis indépendamment l'un de l'autre parmi H, OH, O, NH₂, les halogènes (F, Br, Cl, I), SCH₃, SH, une amine, un amide (dans le sens -NH-CO-R) ou un alkyle linéaire (sens -OR) ou ramifié comportant 1 à 6 atomes de carbone,
Z1 et Z2 étant choisis indépendamment l'un de l'autre parmi H et un groupe organique ;
en tant que substrat d'une polymérase, d'une reverse transcriptase et/ou d'une nucléotide kinase.

2. Utilisation d'un composé de formule générale I : 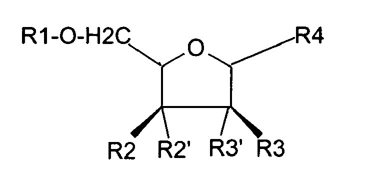 dans laquelle :
- le groupe R1 représente l'hydrogène ou un groupe choisi parmi les groupes phosphate, diphosphate ou triphosphate et les groupes protecteurs tels que DMT.
- R2 et R2' sont choisis indépendamment l'un de l'autre parmi H, OH, le phosphoramidite et ses dérivés, le H-phosphonate et un terminateur d'élongation T,
- R3 et R3' sont choisis indépendamment l'un de l'autre parmi H, OH, les halogènes (F, Br, CI, I), un groupe alkyle linéaire ou ramifié comportant 1 à 6 atomes de carbone et un groupe O-R5, R5 représentant un groupe alkyle linéaire ou ramifié comportant 1 à 6 atomes de carbone,
- R4 représente un hétérocycle (symbolisé par un cercle) substitué, de formule
- 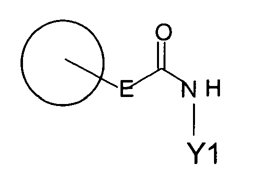 dans laquelle E désigne un bras espaceur constitué par une chaîne carbonée de 0 à 20 atomes de carbones saturée ou non, comprenant ou non des hétéroatomes et/ou éventuellement substitué ; ledit hétérocycle étant le triazole ; 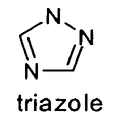
- Y1 représentant NH₂ (désigné par le synthon X₀) ou un groupe : 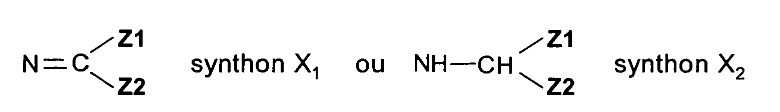
- Y2 et Y3 sont choisis indépendamment l'un de l'autre parmi H, OH, O, NH₂, les halogènes (F, Br, Cl, I), SCH₃, SH, une amine, un amide (dans le sens -NH-CO-R) ou un alkyle linéaire (sens -OR) ou ramifié comportant 1 à 6 atomes de carbone,
Z1 et Z2 étant choisis indépendamment l'un de l'autre parmi H et un groupe organique ;
en tant que substrat d'une polymérase, d'une reverse transcriptase et/ou d'une nucléotide kinase.

3. Utilisation d'un composé de formule (I) selon la revendication 2, **caractérisée en ce que** le groupe R1 est un groupe triphosphate.

4. Utilisation selon l'une quelconque des revendications 1 à 3, **caractérisée en ce qu'**au moins un des groupes R2 et R2' représente un groupe OH.

5. Utilisation selon l'une quelconque des revendications 1 à 4, **caractérisée en ce que** T est sélectionné de préférence parmi F, Br, CI, I, N₃, et un groupe alkyle linéaire ou ramifié comportant 1 à 6 atomes de carbone.

6. Utilisation selon la revendication 1, d'un composé correspondant aux synthons de formule générale II (synthon Xₒ) : 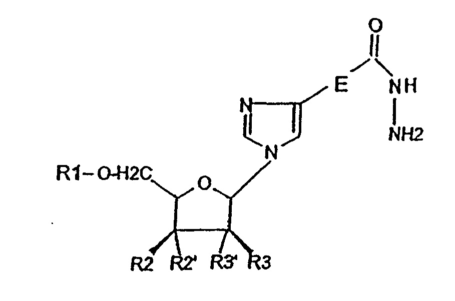 dans lesquels RI, R2, R2', R3, R3'et E correspondent aux groupes de la formule I.

7. Utilisation selon l'une quelconque des revendications 1 à 6, **caractérisée en ce que** Y1 représente un groupe : 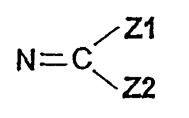 Z1 et Z2 représentant indépendamment l'un de l'autre un hydrogène ou un groupe organique (synthons X₁ et X₁ TP).

8. Utilisation selon l'une quelconque des revendications 1 à 6, **caractérisée en ce que** Y1 représente un groupe : 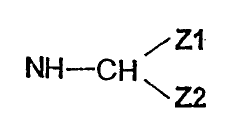 ZI et Z2 représentant indépendamment l'un de l'autre un hydrogène ou un groupe organique (synthons X₂ et X₂TP).

9. Utilisation selon l'une quelconque des revendications 1 à 8, **caractérisée en ce qu'**au moins un des groupes Z1 et Z2 est sélectionné parmi les groupes aromatiques, notamment les groupes de formule:

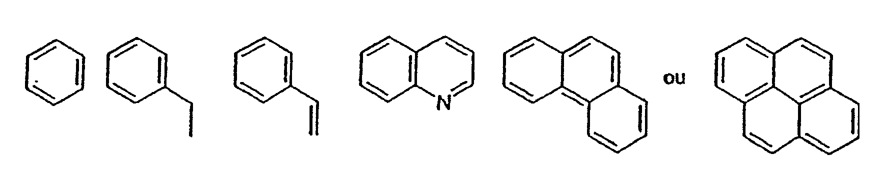

10. Utilisation selon l'une quelconque des revendications 1 à 8, **caractérisée en ce qu'**au moins un des groupes Z1 et Z2 est sélectionné parmi les groupes thiol, alkyle, carbonyle, amine, alcool, aryle, et acide aminé.

11. Utilisation selon l'une quelconque des revendications 1 à 10, **caractérisée en ce que** Z1 ou Z2 forme un cycle avec Y3.

12. Utilisation selon l'une quelconque des revendications 1 à 11, **caractérisée en ce que** ZI ou Z2 comprend un marqueur fluorescent ou phosphorescent, notamment la fluorescamine.

13. Utilisation d'un composé de formule (I) selon l'une quelconque des revendications 1 à 12 pour former des appariements avec les bases naturelles.

14. Utilisation d'un composé selon l'une quelconque des revendications 1 à 11 pour inhiber une polymérase, notamment une reverse transcriptase de rétrovirus et/ou une kinase.

15. Procédé d'identification de composés capables d'introduire une mutation dans un acide nucléique comprenant les étapes consistant à
i) incorporer dans un oligonucléotide synthétique un composé tel que défini dans la revendication 6,
ii) condenser ledit composé avec un aldehyde ou une cétone, éventuellement suivi d'une réduction,
iii) répliquer ledit oligonucléotide à l'aide d'une polymérase et déterminer si une mutation a été introduite dans le brin nouvellement synthétisé.

16. Procédé de mutation ponctuelle d'un acide nucléique, **caractérisé en qu'**on
i) prépare un oligonucléotide synthétique comprenant au moins un composé tel que défini dans les revendications 1 à 12,
ii) on réplique ledit oligonucléotide à l'aide d'une polymérase.

17. Procédé de mutation aléatoire d'un acide nucléique comprenant les étapes consistant à utiliser un mélange réactionnel comprenant au moins un composé tel que défini dans l'une des revendications 1 à 12 pour l'amplification d'un acide nucléique.

18. Procédé selon l'une des revendications 15 à 17, **caractérisé en ce qu'**on utilise une ADN polymérase exo-.

19. Procédé d'identification d'un composé capable d'inhiber une enzyme sélectionnée parmi une polymérase, notamment une réverse transcriptase et une kinase, **caractérisé en ce que** l'on test l'activité de ladite enzyme en présence d'au moins un composé tel que défini dans l'une des revendications 1 à 12.

20. Procédé d'identification d'un composé capable d'inhiber une réverse transcriptase d'un rétrovirus **caractérisé en ce que** l'on test l'effet d'au moins un composé tel que défini dans l'une des revendications 1 à 12, sur des cellules infectées par un rétrovirus, ledit composé se trouvant sous le forme d'un analogue de nucléoside.

21. Acide nucléique comportant au moins un composé tel que défini dans l'une des revendications 1 à 12.

22. Utilisation d'un composé tel que défini dans l'une quelconque des revendications 1 à 12, dans un procédé de mutagénèse aléatoire.

23. Utilisation d'un composé tel que défini dans l'une quelconque des revendications 1 à 12, dans laquelle le dit composé est utilisé comme base ambiguë.

24. Utilisation d'un composé tel que défini dans l'une des revendications 1 à 12, dans une amorce ou une sonde pour l'amplification et/ou la détection d'un acide nucléique cible.

25. Utilisation selon la revendication 24, **caractérisée en ce que** ledit composé est marqué.

26. Kit pour l'amplification et/ou la détection d'un acide nucléique cible comprenant un composé tel que défini dans l'une des revendications 1 à 12, ou un acide nucléique selon la revendication 21.

27. Utilisation d'un acide nucléique selon la revendication 21 pour la fabrication d'un médicament.

28. Utilisation selon la revendication 27, **caractérisée en ce que** ledit acide nucléique est utilisé en tant que ribozyme.

29. Utilisation selon la revendication 27, **caractérisée en ce que** ledit acide nucléique est utilisé en tant qu'antisens.

30. Utilisation d'un composé tel que défini dans la revendication 2, dans lequel YI est NH₂, comme synthon de départ pour la préparation de librairie d'analogues de nucléotides et/ou de nucléosides.

31. Utilisation d'un composé tel que défini dans l'une quelconque des revendications 1 à 12, pour la fabrication d'un médicament.

32. Utilisation d'un composé tel que défini dans l'une quelconque des revendications 1 à 12, pour la fabrication d'un médicament destiné au traitement des infections rétrovirales.

33. Utilisation d'un composé tel que défini dans l'une quelconque des revendications 1 à 11, pour la fabrication d'un médicament destiné au traitement du cancer.
